# FASCICULE DE BREVET EUROPEEN

(11) **EP 4 346 863 B1**
(45) Date de publication et mention de la délivrance du brevet: **18.06.2025**
(21) Numéro de dépôt: 22731266.7
(22) Date de dépôt: 13.05.2022
(51) Int. Cl.: A61K 36/28, A61K 8/36, A61K 8/63, A61K 8/9789, A61Q 19/00, A61Q 19/02

(54) **UTILISATION COSMETIQUE D'UN EXTRAIT HUILEUX DE DRECHES D'IMMORTELLE COMME AGENT ECLAIRCISSANT DE LA PEAU**
KOSMETISCHE VERWENDUNG EINES ÖLIGEN STROHBLUMENEXTRAKT ALS HAUTAUFHELLENDER WIRKSTOFF
COSMETIC USE OF AN OILY EXTRACT OF EVERLASTING DREGS AS SKIN LIGHTENING AGENT

(30) Priorité: 03.06.2021 FR 2105840
(43) Date de publication de la demande: 10.04.2024
(73) Titulaire: LABORATOIRES M&L, 04100 Manosque (FR)
(72) Inventeur: KHAN, Cédric, 04860 PIERREVERT (FR); CENIZO, Valérie, 13650 MEYRARGUES (FR); ROUQUET, Virginie, 04100 MANOSQUE (FR)
(74) Mandataire: Renard, Emmanuelle
(86) Numéro de dépôt international: PCT/FR2022/050914
(87) Numéro de publication internationale: WO 2022/254116

(56) Documents cités:
- FR-A1- 3 079 144
- US-B2- 10 166 412
- US-B2- 9 956 151

## Description

### DOMAINE DE L'INVENTION

La présente invention concerne l'utilisation cosmétique d'un extrait huileux de drèches d'immortelle comme agent éclaircissant de la peau. Elle concerne également une composition cosmétique comprenant, dans un milieu physiologiquement acceptable, un extrait huileux de drèches d'immortelle et de l'hexylrésorcinol, ainsi que les utilisations de cette composition.

### ARRIERE-PLAN DE L'INVENTION

Le mécanisme de pigmentation de la peau est un processus complexe de production de mélanine par les mélanocytes, qui sont des cellules spécialisées situées dans la couche basale de l'épiderme. Ce mécanisme fait intervenir schématiquement les principales étapes suivantes :
Tyrosine ---> Dopa ---> Dopaquinone ---> Dopachrome ---> Mélanine,
catalysées par la tyrosinase, qui est l'enzyme essentielle intervenant dans cette suite de réactions.

La couleur de la peau est principalement déterminée par la nature et la concentration de mélanine produite par les mélanocytes. Ces derniers peuvent être activés par des stimuli internes ou externes à produire de la mélanine dans des organelles spécialisés apparentés aux lysosomes appelés les mélanosomes. Après maturation, les mélanosomes périnucléaires sont transportés le long des microtubules et du cytosquelette d'actine jusqu'à la périphérie des dendrites des mélanocytes. Les mélanosomes, qui contiennent la mélanine, sont ensuite transférés aux kératinocytes adjacents.

Toute altération d'une étape de ce processus complexe peut aboutir à des troubles de la pigmentation de la peau. En particulier, une augmentation du nombre de mélanocytes actifs, et/ou une augmentation de la production de mélanine, et/ou une augmentation du transfert de mélanosomes des mélanocytes vers les kératinocytes peuvent conduire à une hyperpigmentation de la peau.

Ainsi, des facteurs environnementaux, génétiques et/ou hormonaux peuvent moduler la quantité, le type et la distribution de la mélanine dans la peau et/ou les cheveux, avec des conséquences cosmétiques et psychologiques importantes. Des taches plus foncées et/ou plus colorées peuvent ainsi apparaître sur la peau et plus spécialement sur les mains de certaines personnes au soleil. D'autres types d'hyperpigmentations régionales par hyperactivité mélanocytaire sont les mélasmas idiopathiques, survenant lors de la grossesse ("masque de grossesse" ou chloasma) ou d'une contraception oestro-progestative. A l'inverse, une hypopigmentation de la peau peut être observée en cas d'exposition au soleil de cicatrices ou dans le cas de certaines leucodermies telles que le vitiligo. Dans ces situations, à défaut de pouvoir repigmenter la peau lésée, on achève de dépigmenter les zones de peau normale résiduelle pour donner à l'ensemble de la peau une teinte blanche homogène.

L'utilisation de substances dépigmentantes topiques inoffensives présentant une bonne efficacité est tout particulièrement recherchée en vue de traiter toutes ces hyperpigmentations. Une substance est reconnue comme blanchissante ou dépigmentante si elle agit directement sur la vitalité des mélanocytes épidermiques où se déroule la mélanogénèse, et/ou si elle interfère avec une des étapes de la biosynthèse de la mélanine, soit en inhibant une des enzymes impliquées dans la mélanogénèse, en particulier la tyrosinase, soit en s'intercalant comme analogue structural d'un des composés chimiques de la chaîne de synthèse de la mélanine, chaîne qui peut alors être bloquée et ainsi assurer la dépigmentation, et donc l'éclaircissement de la peau.

Pour traiter ces problématiques d'hyperpigmentations, on utilise aujourd'hui divers actifs cosmétiques tels que la vitamine C et ses dérivés, l'arbutine, l'acide azélaïque, l'acide glycolique, le n-butylrésorcinol (ou rucinol), l'hexylrésorcinol, ainsi que le glutathion et ses dérivés. Ces composés présentent différents modes d'action, parmi lesquels l'inhibition de l'activité de la tyrosinase, la réduction de la quantité de mélanine formée ou encore la stimulation de l'élimination de la mélanine au sein des kératinocytes. Certains de ces composés peuvent toutefois entraîner des effets secondaires tels que des brûlures, des érythèmes et une sécheresse de la peau.

II subsiste donc le besoin d'un nouvel agent blanchissant de la peau humaine à action aussi efficace que ceux connus, mais n'ayant pas leurs inconvénients, c'est-à-dire qui soit non irritant, non toxique et/ou non allergisant pour la peau.

Dans ce contexte, il a déjà été proposé d'utiliser différents extraits d'immortelle comme agents blanchissants, notamment un extrait obtenu par extraction, à l'aide d'un solvant tel que l'éthanol, des parties aériennes d*'Helichrysum gymnocephalum* (DC) Humbert. Cet extrait se caractérise par la présence de dérivés monoterpéniques de chalcone ou dihydrochalcone, en particulier de gymnochalcone (FR2970416). On peut également citer le document WO 2007/015232 qui suggère d'utiliser un extrait (non défini) d'*Helichrysum arenarium* dans un procédé d'élimination des cicatrices et de la pigmentation de la peau, ainsi que le document CN111973544 qui fait état de l'effet dépigmentant d'un extrait hydroglycolique d*'Helichrysum bracteatum.* Il a en outre été attribué des propriétés blanchissantes à l'huile essentielle d'*Helichrysum angustifolium* (CN108670926). Il a par ailleurs été suggéré d'utiliser une combinaison de plusieurs extraits différents d'immortelle pour ses propriétés anti-âge, ainsi que sa capacité à atténuer les taches pigmentaires (FR3003167). Enfin, la demande de brevet WO 2019/086602 suggère qu'un extrait à l'eau subcritique des sommités fleuries d*'Helichrysum italicum,* renfermant de l'acide caféoylquinique, pourrait présenter des propriétés dépigmentantes et être utilisé comme actif cosmétique anti-taches.

La Demanderesse a maintenant mis en évidence de manière tout-à-fait surprenante qu'un autre extrait d'immortelle, déjà connu pour ses propriétés de stimulation de la différenciation des kératinocytes et sa capacité à restaurer la barrière cutanée (WO 2019/180368), présentait une bonne activité dépigmentante, même à faible concentration, tout en étant bien toléré.

### RESUME DE L'INVENTION

L'invention a précisément pour objet l'utilisation cosmétique d'un extrait huileux de drèches d'immortelle de l'espèce *Helichrysum italicum,* appliqué topiquement sur la peau, comme agent blanchissant de la peau et/ou éclaircissant du teint et/ou pour homogénéiser la couleur de la peau et/ou réduire ou prévenir l'apparition des taches pigmentaires.

Elle a également pour objet un agent blanchissant de la peau et/ou éclaircissant du teint et/ou pour homogénéiser la couleur de la peau et/ou réduire ou prévenir l'apparition des taches pigmentaires, constitué d'un extrait huileux de drèches d'immortelle de l'espèce *Helichrysum italicum.*

Un autre objet de l'invention porte sur une composition cosmétique comprenant, dans un milieu physiologiquement acceptable, de 0,01 à 1% en poids d'un extrait huileux de drèches d'immortelle tel que défini ci-dessus, et de 0,001 à 5% en poids d'hexylrésorcinol, par rapport au poids total de la composition.

L'invention a encore pour objet l'utilisation cosmétique de la composition cosmétique précitée pour blanchir la peau et/ou éclaircir le teint et/ou pour homogénéiser la couleur de la peau et/ou réduire ou prévenir l'apparition des taches pigmentaires, comprenant l'application topique sur la peau de cette composition.

L'invention a également pour objet un procédé cosmétique pour blanchir la peau et/ou éclaircir le teint et/ou pour homogénéiser la couleur de la peau et/ou réduire ou prévenir l'apparition des taches pigmentaires, comprenant l'application topique sur la peau de la composition précitée.

### DESCRIPTION DETAILLEE

La présente invention met en œuvre un extrait huileux de drèches d'immortelle de l'espèce *Helichrysum italicum,,* préférentiellement d'origine Corse.

Par "drèches", on entend le résidu solide restant après hydrodistillation ou entraînement à la vapeur d'eau de toute partie de l'immortelle et séparation de l'huile essentielle et de l'hydrolat produits. Les drèches d'immortelle utilisées selon l'invention sont donc obtenues suivant un procédé comprenant une étape d'hydrodistillation ou entraînement à la vapeur de tout ou partie de la plante, de préférence des parties aériennes de la plante, en particulier de ses fleurs ou sommités fleuries, suivie d'une étape de récupération du résidu de distillation ou d'entraînement à la vapeur.

L'extrait huileux de drèches peut ensuite être obtenu par tout procédé d'extraction d'huile à partir des drèches, et plus particulièrement par extraction des drèches à l'aide d'un fluide supercritique, tel que le dioxyde de carbone, le monoxyde d'azote, le diméthyléther, le propane, l'éthylène ou le méthanol, sans que cette liste ne soit limitative. On peut ainsi utiliser le dioxyde de carbone à l'état supercritique. L'homme du métier saura ajuster les paramètres d'extraction et notamment la température, la pression et le débit du fluide en fonction de la vitesse d'extraction et du rendement voulus. Par exemple, l'extraction à l'aide de CO₂ supercritique peut être effectuée à une pression de 250 à 300 bars et à une température de 40 à 80°C, de préférence de 50 à 70°C. Après extraction, le fluide supercritique est ramené à l'état gazeux par décompression et généralement recyclé. L'extrait végétal ainsi obtenu est avantageusement ensuite soumis à une étape de distillation moléculaire, par exemple à une température de 180 à 250°C, de préférence de 200 à 220°C, afin de concentrer les insaponifiables présents dans l'extrait. Avant cette étape de distillation moléculaire, l'extrait végétal peut être éventuellement dilué dans un solvant huileux, notamment à base d'huile végétale telle que de l'huile de tournesol. Ce solvant huileux est éliminé lors de l'étape de distillation moléculaire.

Selon une forme d'exécution préférée, l'extrait huileux de drèches d'immortelle utilisé selon l'invention est obtenu suivant un procédé comprenant une étape d'extraction des drèches à l'aide d'un fluide supercritique, de préférence le dioxyde de carbone à l'état supercritique, éventuellement suivie d'une étape de distillation moléculaire.

On obtient ainsi un distillat renfermant notamment de 30 à 50% en poids, plus particulièrement de 35 à 45% en poids, d'insaponifiables et de 1 à 10% en poids, plus particulièrement de 3 à 7% en poids, de stérols, essentiellement du beta-sitostérol.

L'extrait obtenu à l'aide du fluide supercritique ou le distillat résultant de l'étape de distillation moléculaire peut éventuellement être dilué dans un solvant huileux, à une concentration de 1 à 10% en poids, par exemple, notamment de 3 à 5% en poids, afin de faciliter sa manipulation. Tous les solvants huileux utilisables en cosmétique peuvent être utilisés à cette fin, notamment les triglycérides d'acides gras, tels que le triglycéride caprylique / caprique.

La Demanderesse a caractérisé l'extrait huileux de drèches d'immortelle par chromatographie en phase gazeuse couplée à la spectrométrie de masse. Il a ainsi été démontré que l'extrait huileux de drèches d'immortelle renferme moins de 10% de fraction volatile, telle que déterminée par chromatographie en phase gazeuse.

Il a également été démontré que l'extrait huileux de drèches d'immortelle utilisé selon l'invention se différenciait, sur le plan de sa composition, d'un extrait obtenu par CO₂ supercritique de sommités fleuries d'immortelle par le fait qu'il ne renfermait sensiblement pas (c'est-à-dire moins de 10% en poids, voire moins de 5% en poids ou même moins de 2% en poids), voire pas du tout, d'acide palmitoléique, de linalol, de nérol, de 2-décénal, de 2-tridécanone, d'heptadécane, de 8-heptadécène, d'acide isobutyrique, d'acide isovalérique, d'hexadécénol, d'acide laurique, d'acide pentadécanoïque, d'acide décanoïque et d'acide isostéarique. Plus généralement, cet extrait ne renferme pas d'acides gras saturés à plus de 10 atomes de carbone. A contrario, l'extrait huileux de drèches d'immortelle utilisé selon l'invention contient des constituants qui n'ont pas été identifiés dans un extrait obtenu par CO₂ supercritique de sommités fleuries d'immortelle, à savoir l'acide 2(Z)-décénoïque et l'acide 4(Z)-décénoïque.

En outre, par comparaison avec une huile essentielle d'immortelle, l'extrait huileux de drèches d'immortelle utilisé selon l'invention renferme des stérols, essentiellement du beta-sitostérol, des acides carboxyliques linéaires, saturés ou insaturés, en C₇-C₁₀ (en particulier des acides heptanoïque, octénoïque et décénoïque), de l'acide benzoïque et des esters éthyliques d'acides gras linéaires, saturés ou insaturés, en C₈-C₁₈ (en particulier caprylate, caprate et linoléate).

Il se différencie également d'un extrait huileux d'immortelle obtenu par extraction à l'aide d'huile de tournesol en ce qu'il ne renferme pas de limonène et renferme des acides gras et/ou leurs esters éthyliques (acides heptanoïque, 2-octénoïque, 4-octénoique, nonanoïque, 2-décénoïque, 4-décénoïque, linoléique) et de l'acide benzoïque qui ne sont pas présents dans l'extrait huileux précité.

Enfin, l'extrait huileux de drèches d'immortelle utilisé selon l'invention est pauvre en polyphénols et notamment exempt d'acide dicaféoylquinique et de chalcones.

L'extrait huileux de drèches d'immortelle représente avantageusement de 0,01 à 1% du poids total de la composition selon l'invention.

L'extrait huileux de drèches d'immortelle selon l'invention est inclus dans une composition cosmétique qui renferme un milieu physiologiquement acceptable, en particulier cosmétiquement acceptable, c'est-à-dire qui ne génère pas de picotements ou de rougeurs incompatibles avec une utilisation cosmétique. Ce milieu renferme de préférence une phase aqueuse et/ou une phase grasse. On préfère que la composition se présente sous forme d'émulsion, notamment du type huile-dans-eau ou eau-dans-huile, ou d'une composition huileuse anhydre. Par « composition anhydre », on entend une composition renfermant moins de 5% en poids d'eau, de préférence moins de 2% en poids d'eau, voire pas d'eau du tout.

Lorsqu'elle est présente, la phase aqueuse renferme de l'eau et éventuellement au moins un constituant choisi parmi les polyols et les gélifiants aqueux. L'eau représente avantageusement de 40 à 80%, par exemple de 50 à 70%, du poids total de la composition. Le polyol peut notamment être choisi parmi la glycérine, le propylène glycol, le butylène glycol, le pentylène glycol et leurs mélanges et il peut représenter de 5 à 30%, de préférence de 15 à 25%, du poids total de la composition.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les homopolymères d'acrylate de sodium éventuellement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés ; et leurs mélanges.

De son côté, lorsqu'elle est présente, la phase grasse peut comprendre une ou plusieurs huiles volatiles et/ou non volatiles. Des exemples d'huiles volatiles sont les alcanes ramifiés, tels que l'isododécane, et les alcanes linéaires en C₁₀-C₁₃. Comme huiles non volatiles, on peut citer notamment :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides oléique et linoléique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Selon une forme d'exécution préférée, cette composition renferme en outre au moins un actif dépigmentant autre que l'extrait huileux de drèches d'immortelle, qui peut notamment être choisi parmi : des extraits de plantes, en particulier un extrait de réglisse ; la vitamine C et ses dérivés, en particulier l'acide ascorbique, le glucoside d'ascorbyle, l'éther éthylique d'acide ascorbique, le tétraisopalmitate d'ascorbyle et l'ascorbyl phosphate de magnésium ; l'arbutine ; l'acide fértiliqtie ; l'acide kojique ; le résorcinol et ses dérivés tels que l'hexylrésorcinol et le 4-(1-phényléthyl)-1,3-benzenediol commercialisé notamment sous la dénomination commerciale Symwhite 377^{®} par la société Symrise ; l'acide salicylique, ses sels et ses esters ; les extraits de reine des prés et d'*Iris florentina,* le glycyrrhizinate dipotassique ; l'acide tranexamique ; l'acide ellagique ; les dérivés d'acide nicotinique tels que la niacinamide ; l'extrait de kiwi (*Actinidia chinensis*) commercialisé par Gattefossé ; l'extrait de racine de pivoine (*Paeonia suffruticosa*) commercialisé par Ichimaru Pharcos sous la dénomination commerciale Botanpi^{®} Liquid ; le lipoaminoacide (phénylalanine modifiée par l'acide undécylénoïque) commercialisé sous la dénomination commerciale Sepiwhite^{®} par Seppic ; l'huile essentielle d'*Helichrysum italicum* ; et leurs mélanges.

Dans une forme d'exécution préférée de l'invention, la composition cosmétique renferme dans un milieu physiologiquement acceptable, de 0,01 à 1% en poids, de préférence de 0,05 à 1% en poids, et plus préférentiellement de 0,05 à 0,3% en poids, d'un extrait huileux de drèches d'immortelle tel que défini précédemment et de 0,001 à 5% en poids d'hexylrésorcinol, par rapport au poids total de la composition.

Par hexylrésorcinol, ou 4-hexyl-1,3-phénylènediol, on entend le composé de structure chimique :

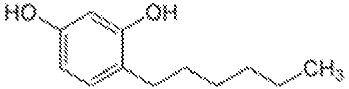

L'hexylrésorcinol est connu pour ses propriétés dépigmentantes et de protection de la peau. De préférence, l'hexylrésorcinol introduit dans la composition cosmétique de l'invention est purifié et ne contient presque pas de résorcinol (ce dernier composé étant instable et ayant un effet irritant pour la peau). Pour ce faire, il est possible d'utiliser le produit obtenu selon le procédé décrit dans le brevet EP 2 152 685 de Sythéon, ou directement le produit commercial Synovea^{®} HR, dont on sait qu'il contient plus de 99,5% d'hexylrésorcinol, qu'il inhibe la production de mélanine in *vitro* (Funasaka AH et al, J. Lipid Res. 1999) et qu'il a une activité blanchissante sur la peau de volontaires humains (EP 2 152 685).

Comme il ressort des exemples ci-après, il a été démontré que l'extrait huileux de drèches d'immortelle selon l'invention potentialise l'effet dépigmentant de l'hexylrésorcinol.

En variante ou en plus, cette composition cosmétique peut comprendre au moins un actif choisi parmi : les agents anti-radicalaires, les agents hydratants, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique et/ou des fibres élastiques; les agents prévenant la dégradation du collagène et/ou des glycosaminoglycanes et/ou des fibrilles d'ancrage dermo-épidermique et/ou des fibres élastiques ; les agents anti-glycation ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl^{®} 3000 et Matrixyl^{®} Synthe'6, respectivement, le palmitoyl tripeptide-8 commercialisé par la société LUCAS MEYER sous la référence commerciale Nutrazen^{®}, le pentapeptide-18 commercialisé par la société LIPOTEC sous la dénomination commerciale Leuphasyl^{®} Solution, le sh-decapeptide-9 commercialisé par la société SANDREAM sous la dénomination commerciale Neoendorphin^{®} et le palmitoyl hexapeptide-52 commercialisé par la société INFINITEC sous la référence commerciale X50 Myocept^{®} Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alpha-hydroxyacides, dont ceux extraits de citron et ceux extraits d'hibiscus commercialisés sous la dénomination commerciale Hibiscus acids ^{®} par Naturex ; les extraits (généralement aqueux) de plantes telles que le trèfle d'eau, la pensée sauvage, la prêle des champs, la Mafane (*Acmella oleracea*), le chardon aux ânes (*Onopordum acanthium*)*,* le millefeuille (*Achillea millefolium,* contenu notamment dans le produit Neurobiox^{®} de la société BASF), l'embelia (*Embelia concinna,* telle que commercialisée par la société SEPPIC), le figuier de Barbarie (*Opuntia ficus indica,* commercialisé notamment par MIBELLE AG BIOCHEMISTRY sous la dénomination commerciale AquaCacteen^{®}), la sauge (*Salvia officinalis,* vendue notamment par PROVITAL GROUP), *Vitex negundo* (commercialisé notamment par les LABORATOIRES EXPANSCIENCE sous la référence commerciale Neurovity^{®}), la châtaigne, la papaye, l'arganier, l'avoine, le tournesol, la pâquerette, la pivoine ou l'aneth ; les extraits aqueux d'algues et notamment de coralline, de janie rouge, *d'Ungaria pinnatifada, d'Alaria esculenta* ou de *Nannochlorosis oculata ;* les huiles essentielles, notamment de myrte ; les gluconates de zinc et/ou de cuivre ; et leurs mélanges.

La composition selon l'invention peut en outre contenir différents constituants qui peuvent être dispersés dans la phase grasse et/ou dans la phase aqueuse, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% et de préférence de 4 à 6% du poids total de la composition.

La composition selon l'invention peut également comprendre une ou plusieurs charges pulvérulentes, qui se présentent avantageusement sous forme de microparticules poreuses ou creuses, de préférence poreuses. Ces microparticules sont en principe sensiblement sphériques. Ces charges peuvent notamment être choisies parmi :
- les charges organiques telles que : les poudres de polysaccharides et en particulier d'amidon natif, d'amidon modifié ou de cellulose ; les poudres de polymères acryliques tels que le poly(méthacrylate de méthyle), de polyamides ou de polyoléfines ; les poudres d'algues séchées telles que *Corallina officinalis ;*
- les charges inorganiques telles que la silice, les argiles, la perlite et le talc ;
- et leurs mélanges.

Comme charge inorganique, on préfère utiliser la silice.

Ces charges peuvent représenter de 1 à 5% en poids, par rapport au poids total de la composition.

La composition selon l'invention peut en outre comprendre des additifs choisis notamment parmi des agents photoprotecteurs organiques et/ou inorganiques, actifs dans la lumière bleue et/ou l'UVA et/ou l'UVB ; des polymères filmogènes à base de polysaccharides, capables de former un film protecteur anti-pollution, tels que les produits commercialisés par SOLABIA sous les dénominations commerciales Pollustop^{®} et Solashield^{®} ; des parfums ; des agents anti-oxydants ; des agents séquestrants ; des ajusteurs de pH ; des conservateurs ; des pigments ; des colorants ; et leurs mélanges.

Cette composition peut se présenter sous toute forme adaptée à une application topique sur la peau et notamment sous forme de lait, de crème, de fluide, de lotion, de gel, de pâte, d'huile ou de film. Il s'agit généralement d'une composition non rincée et en particulier d'une composition de soin de la peau.

En variante, la composition selon l'invention peut être une composition rincée utilisée pour le soin de la peau, en particulier du visage et éventuellement du corps. Dans ce cas, elle peut par exemple être utilisée comme masque ou comme pâte de gommage.

La composition selon l'invention peut être appliquée sur la peau de personnes présentant une inhomogénéité de la couleur de la peau due aux facteurs environnementaux, notamment des taches pigmentaires, un chloasma et/ou des défauts de pigmentation dus à des cicatrices ou des marques d'acné. Elle est généralement appliquée sur au moins une zone du corps concernée et plus particulièrement sur la peau du visage, du décolleté, du cou, des bras, des mains et/ou des jambes, en vue d'éclaircir ces zones et/ou d'uniformiser la couleur de la peau. Cette composition peut être appliquée une ou plusieurs fois par jour, par exemple matin et/ou soir.

En tout état de cause, l'extrait huileux de drèches d'immortelle est appliqué sur la peau en quantité suffisante pour inhiber la mélanogénèse. Par exemple, on peut appliquer chaque jour sur la zone de peau concernée une quantité de 0,5 à 5 mg /cm² , de composition cosmétique renfermant de 0,01 à 1 % en poids d'extrait huileux de drèches d'immortelle selon l'invention.

### FIGURES

La Figure 1 illustre l'effet de l'extrait selon l'invention sur l'inhibition de la production de mélanine par les mélanocytes B16 de souris, par comparaison avec deux contrôles positifs.
La Figure 2 illustre l'effet d'un extrait huileux d'*Helichrysum italicum* sur l'inhibition de la production de mélanine par les mélanocytes B16 de souris, par comparaison avec deux contrôles positifs.
La Figure 3 illustre l'effet d'une combinaison d'un extrait selon l'invention avec l'hexylrésorcinol sur l'inhibition de la production de mélanine par les mélanocytes B16 de souris, par comparaison avec chacun de ces deux actifs et avec deux contrôles positifs.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Préparation et caractérisation de l'extrait - Comparaison avec un extrait huileux de sommités fleuries

### 1.1 - Préparation des extraits

On a produit une huile essentielle et un hydrolat par hydrodistillation des sommités fleuries d'*Helichrysum italicum,* puis le résidu d'hydrodistillation (drèches) a été isolé et extrait à l'aide de CO₂ supercritique à une température de 60 °C et sous une pression de 285 bars. Les insaponifiables ainsi obtenus ont été dilués dans l'huile de tournesol, puis soumis à une étape de distillation moléculaire à 200°C et le distillat résultant a été standardisé en le diluant à hauteur de 3-5% en poids dans des triglycérides caprylique / caprique.

On a comparé la composition en polyphénols de cet extrait avec celle d'un extrait huileux d'*Helichrysum italicum* obtenu comme suit : on a mélangé 9% en poids de poudre de plante (sommités fleuries) séchée et broyée avec 91% d'une huile de tournesol désodorisée à l'aide d'ultrasons, dans un récipient refroidi à 10°C. L'extraction a été réalisée pendant 30 min, sous ultrasons, avec une agitation de 500 tours/min. L'extraction a été poursuivie au micro-ondes sous un flux d'azote de 0,4 L/min. Les phases solide et liquide huileuse obtenues ont été séparées par décantation, puis la phase huileuse a été filtrée et stockée sous atmosphère inerte, à l'abri de la lumière.

### 1.2 - Caractérisation des polyphénols

### Matériel

- Chromatographie liquide ultra haute performance (Agilent Technologies, USA)
- Injecteur automatique 1290 series
- Détecteur à barrette de diodes (DAD) 1260 series
- Spectromètre de Masse : Esquire 6000 (Bruker Daltonics, Bremen) équipé d'une source d'ionisation Electrospray (ESI).

### Préparation des échantillons

L'extrait selon l'invention été préparé par dissolution de 2g d'extrait de drèches dans 1mL d'hexane. Cette solution huileuse a été extraite 3 fois par 2mL d'un mélange MeOH/H₂O (60/40). Les phases MeOH/H₂O ont ensuite été regroupées et diluées au ½ dans un mélange MeOH/H₂O (60/40).

### Méthode d'analyse

Les solutions obtenues après dilution/extraction ont été filtrées sur filtre PTFE (0,45 µm) puis injectées (1µL) sur une colonne Agilent C18 (2,1 mm x 100 mm ; 1,8 µm) à une température maintenue à 25°C et à un débit de 0,4 mL/min. On a utilisé comme solvants : A = H₂O/HCOOH (0,1%) et B = ACN/HCOOH (0,1%). Le gradient d'élution des polyphénols était le suivant :

**[Tableau 1]**

| **Temps (min.)** | **% Solvant B** |
|---|---|
| 0 | 5 |
| 2 | 18,3 |
| 6 | 29 |
| 6,5 | 31,7 |
| 9,5 | 38,3 |
| 11,5 | 71 |
| 15 | 95 |
| 16 | 100 |
| 17 | 100 |
| 17,2 | 5 |
| 20,2 | 5 |

A la sortie du détecteur à barrette de diode, l'éluant a été injecté dans le spectromètre de masse. Les analyses ont été réalisées en mode négatif ou positif.

Les spectres de LC-MS ont été acquis sur l'ensemble de la gamme des masses (m/z) allant de 100 à 1400. L'ensemble des données a ensuite été collecté et traité par le logiciel Hystar version 3.0.

### Résultats

**[Tableau 2]**

| Composé | **Extrait de drèches** |
|---|---|
| Isomère d'acide chlorogénique | n.d. |
| Acide chlorogénique | n.d. |
| Isomère d'acide chlorogénique | n.d. |
| Acide dicaféoyl glucarique | n.d. |
| Acide caféique | n.d. |
| Acide dicaféoyl glucarique | n.d. |
| Quercetagétine-O-hexoside | n.d. |
| Flavonoïde-O-hexoside | n.d. |
| Acide dicaféoyl quinique | n.d. |
| Acide dicaféovl quinique | n.d. |
| Acide dicaféoyl quinique | n.d. |
| Acide dicaféoyl quinique | n.d. |
| Acide dicaféoyl quinique | n.d. |
| Quercétine-O-hexoside-O-rhamnoside | n.d. |
| Arzanol | 37,7 ± 1,3 |
| Méthylarzanol | 2,4 ± 0,3 |
| Diméthylarzanol | n.d. |
| 12-hydroxytrémétone | Présence |
| Gnafaliol | Présence |

| | |
|---|---|
| n.d : non détecté ; <LQ : détecté mais inférieur à la limite de quantification | |

### 1.3 - Caractérisation des chalcones

On a évalué la présence de quatre chalcones (lindératine, linderachalcone, méthyl-lindératine, gymnochalcone) dans l'extrait de drèches décrit ci-dessus, selon une méthode de RMN 2D à spectre HSQC ¹H / ¹³C.

**Matériel et méthode** : Un aliquot de l'extrait de drèches (15mg) a été dissous dans 700 µL de DMSO-d6 afin d'être analysé en RMN 2D à 298 Kelvin.

L'analyse RMN 2D a été réalisée à l'aide d'un spectromètre Bruker Avance AVIII-600 équipé de la sonde RMN Cryoprobe^{®}. Les valeurs obtenues par RMN ont été comparées avec celles calculées à l'aide du logiciel ACD Labs.

**Résultat** : il n'a pas été détecté de chalcones dans l'échantillon.

### 1.4 - Caractérisation des autres constituants

Parallèlement, on a évalué la présence de différents composés organiques dans les deux extraits ci-dessus, ainsi que dans un extrait obtenu de manière similaire à l'extrait selon l'invention, mais suivant un procédé mis en oeuvre sur les sommités fleuries d'immortelle et non sur leur résidu d'hydrodistillation.

### Matériel

- Chromatographie gazeuse Trace GC Ultra (ThermoElectron SAS)
- Spectromètre de Masse : ISQ MASS SPECTROMETER EI LT LARGE T (ThermoElectron SAS) utilisé en mode EI

### Préparation des échantillons

Les échantillons ont été préparés par dilution de 0,3g d'extrait dans 6mL d'isooctane. 0,6mL de potasse méthanolique sont ajoutés et le mélange est agité 30s au vortex.

1g de NaHSO₄ est ajouté et le mélange est agité 30s au vortex.

Le mélange est ensuite mis à décanter.

La partie supérieure du surnageant est prélevée pour analyse chromatographique.

### Méthode d'analyse

Les échantillons sont analysés selon les conditions suivantes :
Colonne chromatographique polaire de type TR WAX 60m x 0.25µm x 0.25mm
Débit d'H₂ : 1mL/min
Programmation du Four : 50°C (2min) puis 10°C/min jusqu'à 250°C (18min)
Split ratio : 100
Volume d'injection : 1µL
Température de l'injecteur : 250°C
Mass range : 50-650 amu

### Résultats

Il a notamment été observé que l'extrait de drèches d'immortelle selon l'invention se différencie de l'extrait huileux de sommités fleuries en ce qu'il ne renferme pas de limonène, contient beaucoup plus de β-eudesmol et renferme des acides gras et/ou leurs esters éthyliques (acides heptanoïque, 2-octénoïque, 4-octénoique, nonanoïque, 2-décénoïque, 4-décénoïque, linoléique) et de l'acide benzoïque qui ne sont pas présents dans l'extrait huileux de sommités fleuries d'immortelle.

En outre, l'extrait de drèches selon l'invention se différencie notamment de l'extrait au CO₂ supercritique de sommités fleuries en ce qu'il ne renferme pas d'acides gras saturés de plus de 10 atomes de carbone et en ce qu'il renferme beaucoup moins, voire pas du tout, de terpénoïdes (mono-terpènes et sesquiterpènes notamment), en particulier en ce qu'il est dépourvu de nérol et de linalol, et renferme beaucoup moins, voire pas du tout, d'alcanes lourds (renfermant plus de 16 atomes de carbone), et est dépourvu d'acides palmitoléique, isobutyrique et isovalérique, de 2-décénal et d'hexadécénol. L'extrait de drèches selon l'invention renferme par ailleurs des molécules caractéristiques qui ne sont pas présentes dans l'extrait au CO₂ supercritique de sommités fleuries, à savoir les acides (2)-(Z)-décénoïque et 4-(Z)-décénoïque.

### Exemple 2 : Effet de l'extrait de drèches d'immortelle selon l'invention sur la mélanogénèse

L'extrait de drèches d'immortelle et l'extrait huileux de sommités fleuries décrits à l'Exemple 1 ont été testés pour évaluer leur capacité à inhiber la production de mélanine.

### 2.1 - Matériels & Méthode

Des mélanocytes (lignée B16 de souris) sont ensemencés en plaque 96 puits à raison de 2000 cellules par puits en milieu DMEM sans rouge de phénol contenant 10% de sérum de veau fœtal, 2mM de glutamine, 1 mM de pyruvate de sodium et 100 µg/ml de Normocine. Le lendemain, les cellules sont traitées par addition de 0,1 µM de NDP-MSH pour induire la pigmentation. Dans les conditions de test, en présence de NDP-MSH, l'acide kojique à 3mM et la vitamine C (ascorbyl glucoside) à 0,5% sont utilisés comme témoins positifs d'inhibition de la pigmentation et les extraits à évaluer sont testés à plusieurs concentrations. Toutes les conditions sont testées en triplicat. Les cellules sont incubées à 37°C 5% de CO2 durant 72h en présence des extraits et témoins positifs. Puis la viabilité cellulaire est évaluée par la méthode du XTT et seules les conditions non-cytotoxiques (dont la viabilité est supérieure à 85% de celle du contrôle non traité) sont analysées pour la quantité de mélanine.

Pour le dosage de mélanine, le milieu de chaque puits est aspiré et les cellules lysées par ajout de 50 µl de NaOH 1M sur le tapis cellulaire. Les plaques sont agitées 30 minutes à température ambiante puis le lysat est transféré en flacons de verre et incubé 1 nuit à 95°C. après incubation, les lysats sont centrifugés à 2000 tours/min pour éliminer les débris cellulaires. L'absorbance du surnageant est lue à 405 nm et reflète la quantité de mélanine de chaque puits et la moyenne des 3 réplicats est réalisée pour chaque condition. Un puits de cellules non traitées à la NDP-MSH est utilisé comme témoin et sa valeur de DO soustraite à toutes les valeurs de DO des autres puits. Puis le pourcentage d'inhibition de la mélanine est calculé selon la formule : 100- ((quantité de mélanine de la condition test/ quantité de mélanine du témoin non traité) x100)

La significativité des résultats est évaluée au moyen du test Anova à un facteur (pour l'extrait de drèches et l'extrait huileux de sommités fleuries) ou du test de Student (pour l'acide kojique et la vitamine C).

### 2.2 - Résultats

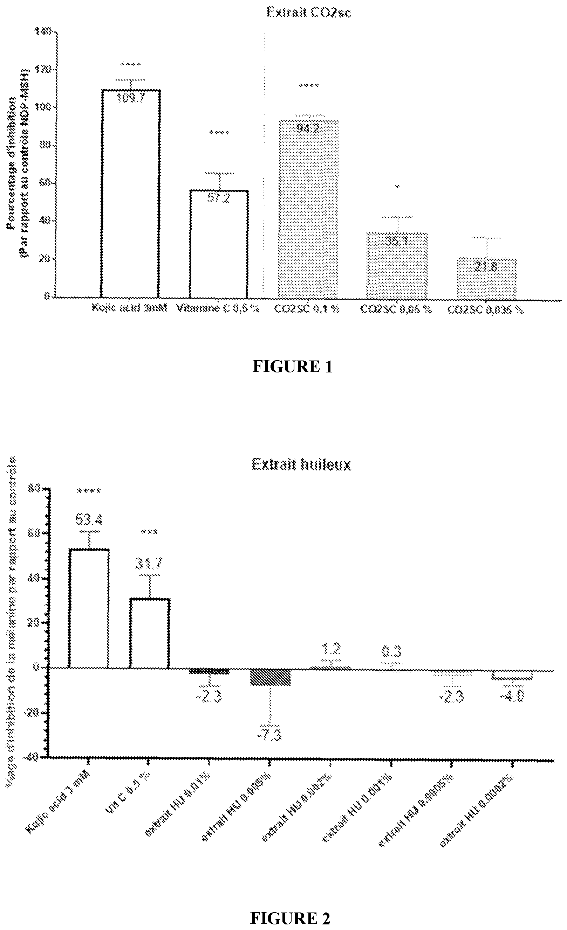

Comme illustré par les Figures 1 et 2, le test est validé par mise en évidence de l'efficacité de l'acide kojique et de la vitamine C (***p<0,001 ; ****p<0,0001).

En outre, la Figure 1 montre que l'extrait de drèches d'immortelle selon l'invention inhibe la pigmentation des mélanocytes de manière significative et de manière dose-dépendante à partir d'une concentration de 0,05% (****p<0,0001 à 0,1% ; *p<0,05 à 0,05%). A une concentration de 0,1%, l'extrait de drèches selon l'invention présente une efficacité bien supérieure à la vitamine C utilisée à plus forte concentration, qui constitue l'actif blanchissant classiquement utilisé en cosmétique. Son efficacité est proche de celle de l'acide kojique qui est un dépigmentant de référence, connu toutefois pour entraîner des risques d'allergie et d'irritation sévères.

Cet exemple démontre ainsi que l'extrait de drèches d'immortelle selon l'invention constitue un actif cosmétique de choix pour éclaircir la peau.

Par comparaison, la Figure 2 montre que l'effet sur la pigmentation de l'extrait huileux de sommités fleuries d'immortelle n'est pas significativement différent du témoin.

### Exemple 3 : Effet d'une combinaison d'hexylrésorcinol et d'un extrait de drèches d'immortelle selon l'invention sur la mélanogénèse

Le test décrit à l'Exemple 2 a été reproduit en ajoutant à l'extrait de drèches d'immortelle selon l'invention de l'hexylrésorcinol. Une étude préalable de cytotoxicité a permis de définir les doses maximales non cytotoxiques pouvant être appliquées sur les cellules, à savoir de 10 µg/mL pour l'hexylrésorcinol et de 1% pour l'extrait de drèches d'immortelle selon l'invention. Ces deux actifs ont été testés à une concentration de 0,035% pour l'extrait de drèches d'immortelle et de 5 µg/mL pour l'hexylrésorcinol.

Les résultats ont été comparés à l'effet obtenu avec l'extrait selon l'invention seul et l'hexylrésorcinol seul, utilisés aux mêmes doses.

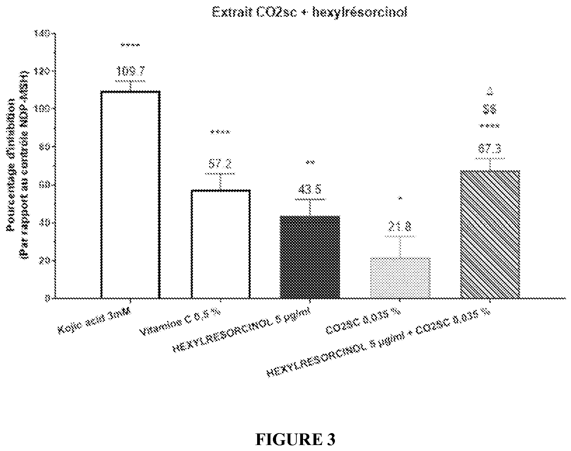

Comme le montre la Figure 3, le test est validé par mise en évidence de l'efficacité de l'acide kojique et de la vitamine C (****p<0,0001) et confirme l'efficacité dépigmentante de l'hexylrésorcinol.

En outre, la combinaison testée inhibe plus fortement la pigmentation que lorsque les deux actifs sont utilisés seuls. Cette efficacité est significativement différente du contrôle non traité mais également de chaque actif utilisé seul à la même concentration (Test Anova à un facteur, $$ p<0,01 vs. extrait de drèches ; Δ p<0,05 vs. hexylrésorcinol).

Cet essai démontre ainsi que l'effet de l'hexylrésorcinol sur la mélanogénèse est significativement potentialisé par addition de l'extrait selon l'invention, ce qui n'était pas prévisible.

### Exemple 4 : Compositions cosmétiques

Les composition suivantes sont préparées de manière classique pour l'homme de l'art, en mélangeant les ingrédients ci-dessous dans les proportions pondérales indiquées.

### Crème Mains

| | |
|---|---|
| Huiles | 10-15% |
| Polyols | 20% |
| Beurre de karité | 5 % |
| Tensioactifs non ioniques | 3-5% |
| Co-émulsionnant | 1-5-2% |
| Charges pulvérulentes | 2-5% |
| Gélifiants aqueux | 1-2% |
| Hexylrésorcinol | 0,5 % |
| Extrait selon l'invention | 0,1% |
| Huile essentielle d'immortelle | 0,001% |
| Adénosine | 0,04% |
| Anti-oxydant | qs |
| Parfum | qs |
| Eau | qsp 100% |

### Huile visage et corps

### Huiles

| | |
|---|---|
| Esters d'acides gras | 50-60% |
| Huiles végétales | 15-20% |
| Alcanes | 10-15% |
| Alcools gras ramifiés | 10-20% |
| Extrait selon l'invention | 0,05% |
| Anti-oxydant | qs |
| Parfum | qs |

## Revendications

1. Utilisation cosmétique d'un extrait huileux de drèches d'immortelle de l'espèce *Helichrysum italicum,* appliqué topiquement sur la peau, comme agent blanchissant de la peau et/ou éclaircissant du teint et/ou pour homogénéiser la couleur de la peau et/ou réduire ou prévenir l'apparition des taches pigmentaires.

2. Utilisation selon la revendication 1, **caractérisée en ce que** l'extrait huileux de drèches d'immortelle renferme moins de 10% de fraction volatile, telle que déterminée par chromatographie en phase gazeuse.

3. Utilisation selon la revendication 1 ou 2, **caractérisée en ce que** l'extrait huileux de drèches d'immortelle renferme des acides carboxyliques linéaires, saturés ou insaturés, en C₇-C₁₀, y compris l'acide 2-(Z)-décénoïque et l'acide 4-(Z)-décénoïque et de préférence les acides heptanoïque, octénoïque et décénoïque ; et des esters éthyliques d'acides gras linéaires, saturés ou insaturés, en C₈-C₁₈, de préférence les caprylate, caprate, et linoléate d'éthyle.

4. Utilisation selon l'une quelconque des revendications 1 à 3, **caractérisée en ce que** l'extrait huileux de drèches d'immortelle renferme des stérols, qui sont de préférence majoritairement constitués du beta-sitostérol.

5. Utilisation selon l'une quelconque des revendications 1 à 4, **caractérisée en ce que** l'immortelle de l'espèce d'*Helichrysum italicum* est d'origine Corse.

6. Utilisation selon l'une quelconque des revendications 1 à 5, **caractérisée en ce que** l'extrait huileux de drèches d'immortelle est obtenu suivant un procédé comprenant une étape d'extraction des drèches à l'aide d'un fluide supercritique, de préférence le dioxyde de carbone à l'état supercritique, éventuellement suivie d'une étape de distillation moléculaire.

7. Utilisation selon la revendication 6, **caractérisée en ce que** l'extrait huileux de drèches d'immortelle obtenu par extraction et éventuellement distillation moléculaire est dilué dans un solvant huileux, tel que les triglycérides d'acide caprylique / caprique.

8. Utilisation selon la revendication 6, **caractérisée en ce que** les drèches d'immortelle sont obtenues suivant un procédé comprenant une étape d'hydrodistillation ou entraînement à la vapeur de tout ou partie de la plante, de préférence des parties aériennes de la plante, en particulier de ses fleurs ou sommités fleuries, suivie d'une étape de récupération du résidu de distillation ou d'entraînement à la vapeur.

9. Utilisation selon l'une quelconque des revendications 1 à 8, **caractérisée en ce que** l'extrait huileux de drèches d'immortelle est appliqué sur la peau du visage, du décolleté, du cou, des bras, des mains et/ou des jambes.

10. Utilisation selon l'une quelconque des revendications 1 à 9, **caractérisée en ce que** l'extrait huileux de drèches d'immortelle est appliqué sur la peau en quantité suffisante pour inhiber la mélanogénèse.

11. Utilisation selon l'une quelconque des revendications 1 à 10, **caractérisée en ce que** l'extrait huileux de drèches d'immortelle est appliqué sur la peau de personnes présentant une inhomogénéité de la couleur de la peau due aux facteurs environnementaux, notamment des taches pigmentaires, un chloasma et/ou des défauts de pigmentation dus à des cicatrices ou des marques d'acné.

12. Composition cosmétique comprenant, dans un milieu physiologiquement acceptable, de 0,01 à 1% en poids d'un extrait huileux de drèches d'immortelle tel que défini dans l'une quelconque des revendications 1 à 8, et de 0,001 à 5% en poids d'hexylrésorcinol, par rapport au poids total de la composition.

13. Utilisation cosmétique de la composition selon la revendication 12, pour blanchir la peau et/ou éclaircir le teint et/ou pour homogénéiser la couleur de la peau et/ou réduire ou prévenir l'apparition des taches pigmentaires, comprenant l'application topique sur la peau de cette composition.

14. Agent blanchissant de la peau et/ou éclaircissant du teint et/ou pour homogénéiser la couleur de la peau et/ou réduire ou prévenir l'apparition des taches pigmentaires, constitué d'un extrait huileux de drèches d'immortelle de l'espèce *Helichrysum italicum.*

15. Procédé cosmétique pour blanchir la peau et/ou éclaircir le teint et/ou pour homogénéiser la couleur de la peau et/ou réduire ou prévenir l'apparition des taches pigmentaires, comprenant l'application topique sur la peau de la composition selon la revendication 12.

## Patentansprüche

1. Kosmetische Verwendung eines öligen Extrakts von Rückständen einer Destillation einer Immortelle der Spezies *Helichrysum italicum,* der topisch auf die Haut aufgetragen wird, als Mittel zum Bleichen der Haut und/oder zur Aufhellung des Teints und/oder zur Angleichung der Hautfarbe und/oder zur Verminderung oder Prävention des Auftretens von Pigmentflecken.

2. Verwendung nach Anspruch 1, **dadurch gekennzeichnet, dass** der ölige Extrakt von Immortelle-Destillationsrückständen weniger als 10 % der flüchtigen Fraktion enthält, bestimmt mittels Gaschromatographie.

3. Verwendung nach Anspruch 1 oder 2, **dadurch gekennzeichnet, dass** der ölige Extrakt von Immortelle-Destillationsrückständen gesättigte oder ungesättigte lineare C₇-C₁₀-Carbonsäuren einschließlich 2-(Z)-Decensäure und 4-(Z)-Decensäure und vorzugsweise Heptan-, Octen- und Decensäure und Ethylester von gesättigten oder ungesättigten linearen C₈-C₁₈-Fettsäuren, vorzugsweise Ethylcaprylat, -caprat und linoleat, enthält.

4. Verwendung nach einem der Ansprüche 1 bis 3, **dadurch gekennzeichnet, dass** der ölige Extrakt von Immortelle-Destillationsrückständen Sterine enthält, die vorzugsweise überwiegend aus Beta-Sitosterin bestehen.

5. Verwendung nach einem der Ansprüche 1 bis 4, **dadurch gekennzeichnet, dass** die Immortelle der Spezies *Helichrysum italicum* korsischen Ursprungs ist.

6. Verwendung nach einem der Ansprüche 1 bis 5, **dadurch gekennzeichnet, dass** der ölige Extrakt von Immortelle-Destillationsrückständen nach einem Verfahren erhalten wird, das einen Schritt einer Extraktion von Destillationsrückständen mittels eines überkritischen Fluids, vorzugsweise Kohlendioxid im überkritischen Zustand, umfasst, gegebenenfalls gefolgt von einem Molekulardestillationsschritt.

7. Verwendung nach Anspruch 6, **dadurch gekennzeichnet, dass** der durch eine Extraktion und gegebenenfalls eine Molekulardestillation erhaltene ölige Extrakt von Immortelle-Destillationsrückständen in einem öligen Lösungsmittel, wie Capryl-/Caprinsäuretriglyceriden, verdünnt ist.

8. Verwendung nach Anspruch 6, **dadurch gekennzeichnet, dass** die Immortelle-Destillationsrückstände nach einem Verfahren erhalten werden, das einen Wasserdampfdestillations- oder Dampfdestillationsschritt der gesamten Pflanze oder eines Teils davon, vorzugsweise von oberirdischen Teilen der Pflanze, insbesondere ihrer Blüten oder Stängelspitzen mit Blüten, gefolgt von einem Schritt des Isolierens des Destillations- oder Dampfdestillationsrückstands, umfasst.

9. Verwendung nach einem der Ansprüche 1 bis 8, **dadurch gekennzeichnet, dass** der ölige Extrakt von Immortelle-Destillationsrückständen auf die Haut des Gesichts, des Dekolletés, des Halses, der Arme, der Hände und/oder der Beine aufgetragen wird.

10. Verwendung nach einem der Ansprüche 1 bis 9, **dadurch gekennzeichnet, dass** der ölige Extrakt von Immortelle-Destillationsrückständen in einer Menge auf die Haut aufgetragen wird, die zur Hemmung der Melanogenese ausreichend ist.

11. Verwendung nach einem der Ansprüche 1 bis 10, **dadurch gekennzeichnet, dass** der ölige Extrakt von Immortelle-Destillationsrückständen auf die Haut von Personen aufgetragen wird, die eine Inhomogenität der Hautfarbe aufgrund von Umwelteinflüssen, insbesondere Pigmentflecke, ein Chloasma und/oder Pigmentstörungen aufgrund von Narben oder Aknenarben, aufweisen.

12. Kosmetische Zusammensetzung, die in einem physiologisch annehmbaren Medium 0,01 bis 1 Gew.-% eines öligen Extrakts von Immortelle-Destillationsrückständen gemäß der Definition in einem der Ansprüche 1 bis 8 und 0,001 bis 5 Gew.-% Hexylresorcin, bezogen auf das Gesamtgewicht der Zusammensetzung, umfasst.

13. Kosmetische Verwendung der Zusammensetzung nach Anspruch 12 zum Bleichen der Haut und/oder zur Aufhellung des Teints und/oder zur Angleichung der Hautfarbe und/oder zur Verminderung oder Prävention des Auftretens von Pigmentflecken, welche die topische Anwendung dieser Zusammensetzung auf die Haut umfasst.

14. Mittel zum Bleichen der Haut und/oder zur Aufhellung des Teints und/oder zur Angleichung der Hautfarbe und/oder zur Verminderung oder Prävention des Auftretens von Pigmentflecken, das aus einem öligen Extrakt von Rückständen einer Destillation einer Immortelle der Spezies *Helichrysum italicum* besteht.

15. Kosmetisches Verfahren zum Bleichen der Haut und/oder zur Aufhellung des Teints und/oder zur Angleichung der Hautfarbe und/oder zur Verminderung oder Prävention des Auftretens von Pigmentflecken, das die topische Anwendung der Zusammensetzung nach Anspruch 12 auf die Haut umfasst.

## Claims

1. The cosmetic use of an oily extract of immortelle distillation residue of the *Helichrysum italicum* species, applied topically to the skin, as an agent for bleaching the skin and/or lightening the complexion and/or for homogenizing skin color and/or reducing or preventing the appearance of pigment spots.

2. The use as claimed in claim 1, **characterized in that** the oily extract of immortelle distillation residue contains less than 10% volatile fraction, as determined by gas chromatography.

3. The use as claimed in claim 1 or 2, **characterized in that** the oily extract of immortelle distillation residue contains linear, saturated or unsaturated C₇-C₁₀ carboxylic acids, including 2-(Z)-decenoic acid and 4-(Z)-decenoic acid and preferably heptanoic, octenoic and decenoic acids; and ethyl esters of linear, saturated or unsaturated C₈-C₁₈ fatty acids, preferably ethyl caprylate, caprate and linoleate.

4. The use as claimed in any one of claims 1 to 3, **characterized in that** the oily extract of immortelle distillation residue contains sterols, which are preferably predominantly of beta-sitosterol.

5. The use as claimed in any one of claims 1 to 4, **characterized in that** the immortelle of the *Helichrysum italicum* species is of Corsican origin.

6. The use as claimed in any one of claims 1 to 5, **characterized in that** the oily extract of immortelle distillation residue is obtained according to a process comprising a step of extracting the distillation residue with a supercritical fluid, preferably carbon dioxide in the supercritical state, optionally followed by a molecular distillation step.

7. The use as claimed in claim 6, **characterized in that** the oily extract of immortelle distillation residue obtained by extraction and optionally molecular distillation is diluted in an oily solvent, such as caprylic/capric acid triglycerides.

8. The use as claimed in claim 6, **characterized in that** the immortelle distillation residue are obtained according to a process comprising a step of hydrodistillation or steam distillation of all or part of the plant, preferably the aerial parts of the plant, in particular its flowers or flowering tops, followed by a step of recovery of the distillation or steam distillation residue.

9. The use as claimed in any one of claims 1 to 8, **characterized in that** the oily extract of immortelle distillation residue is applied to the skin of the face, neckline, neck, arms, hands and/or legs.

10. The use as claimed in any one of claims 1 to 9, **characterized in that** the oily extract of immortelle distillation residue is applied to the skin in an amount sufficient to inhibit melanogenesis.

11. The use as claimed in any one of claims 1 to 10, **characterized in that** the oily extract of immortelle distillation residue is applied to the skin of people with skin color inhomogeneity mediated by environmental factors, notably pigment spots, chloasma and/or pigmentation defects due to scars or acne marks.

12. A cosmetic composition comprising, in a physiologically acceptable medium, from 0.01% to 1% by weight of an oily extract of immortelle distillation residue as defined in any one of claims 1 to 8, and from 0.001% to 5% by weight of hexylresorcinol, relative to the total weight of the composition.

13. The cosmetic use of the composition as claimed in claim 12, for bleaching the skin and/or lightening the complexion and/or for homogenizing the skin color and/or reducing or preventing the appearance of pigment spots, comprising the topical application of said composition to the skin.

14. An agent for bleaching the skin and/or lightening the complexion and/or for homogenizing the skin color and/or reducing or preventing the appearance of pigment spots, consisting of an oily extract of immortelle distillation residue of the *Helichrysum italicum* species.

15. A cosmetic process for bleaching the skin and/or lightening the complexion and/or for homogenizing the skin color and/or reducing or preventing the appearance of pigment spots, comprising the topical application to the skin of the composition as claimed in claim 12.
